(19) **Europäisches Patentamt / European Patent Office / Office européen des brevets**

(11) **EP 1 799 105 B1**

(12) **EUROPEAN PATENT SPECIFICATION**

(45) Date of publication and mention
of the grant of the patent:
**29.10.2008 Bulletin 2008/44**

(21) Application number: **04761795.6**

(22) Date of filing: **03.09.2004**

(51) Int Cl.:
*A61B 5/16* (2006.01)     *A61B 3/113* (2006.01)

(86) International application number:
**PCT/CA2004/001632**

(87) International publication number:
**WO 2006/024129 (09.03.2006 Gazette 2006/10)**

(54) **SYSTEM AND METHOD FOR MENTAL WORKLOAD MEASUREMENT BASED ON RAPID EYE MOVEMENT**

SYSTEM UND VERFAHREN ZUR MESSUNG DER GEISTIGEN ARBEITSBELASTUNG AUF GRUNDLAGE RASCHER AUGENBEWEGUNGEN

SYSTEME ET PROCEDE DE MESURE DE LA CHARGE DE TRAVAIL MENTAL SUR LA BASE D'UN MOUVEMENT OCULAIRE RAPIDE

(84) Designated Contracting States:
**AT BE BG CH CY CZ DE DK EE ES FI FR GB GR HU IE IT LI LU MC NL PL PT RO SE SI SK TR**

(43) Date of publication of application:
**27.06.2007 Bulletin 2007/26**

(73) Proprietor: **CANADIAN SPACE AGENCY**
**Saint-Hubert,**
**Quebec J3Y 8Y9 (CA)**

(72) Inventors:
- **BEDZIOUK, Serguei**
  **Ile des Soeurs, Quebec H3E 1T4 (CA)**
- **KOSTIN, Anatoly**
  **Moscow Region, 141700 (RU)**
- **GOLIKOV, Yurij**
  **Moscow, 127577 (RU)**

(74) Representative: **Henrion, Oliver et al**
**Patentanwälte**
**Zellentin & Partner**
**Rubensstrasse 30**
**67061 Ludwigshafen (DE)**

(56) References cited:
| | |
|---|---|
| JP-A- 7 108 038 | US-A- 5 467 777 |
| US-A- 5 649 061 | US-A- 6 090 051 |
| US-A- 6 102 870 | |

- TERAO ET AL: 'Engagement of gaze in capturing targets for future sequential manual actions.' J NEUROPHYSIOL vol. 88, no. 4, October 2002, pages 1716 - 1725, XP008092229
- SIMON ET AL: 'Quantitative analysis of mental workload influence on eye scanning movements' CONFERENCE PROCEEDINGS vol. 4, October 1993, pages 707 - 712, XP000467646
- TAKAHASI ET AL: 'Neural networks for human cognitive state estimation' PROCEEDINGS OF THE IEEE/RS/GI INTERNATIONAL CONFERENCE vol. 3, no. 12, September 1994, pages 2176 - 2183, XP008092571
- MCPEEK ET AL: 'Concurrent processing of saccades in visual search.' VISION SCIENCES LABORATORY vol. 40, no. 18, 2000, pages 2499 - 2516, XP008092008
- MAC KEBEN ET AL: 'Eye movement control during single word reading in dyslexics' J VIS vol. 14, no. 4-5, May 2004, pages 388 - 402, XP008092813
- VERGILLINO ET AL: 'Referenceframes in reading: evidence from visually and memory guided saccades' LABPRATOIRE DE PSYCOLOGIE EXPERIMENTALE vol. 41, no. 25-26, 2001, pages 3547 - 3557, XP008091539
- COLLINS ET AL: 'Timing variability of repetitive saccade eye movements' EXP BRAIN RES vol. 120, no. 3, May 1998, pages 325 - 334, XP008091370

**Description**

**FIELD OF THE INVENTION**

The present invention relates generally to mental workload measurement and eye movement research. More particularly, the present invention relates to mental workload measurement, such as for operator training, based on rapid eye movement.

**BACKGROUND OF THE INVENTION**

It is well known to psychologists and to the general public that simple tasks generally require less concentration and mental effort than difficult tasks. There have been some efforts to attempt to objectively measure this mental effort, sometimes referred to as a psycho-regulation level (from a psychological standpoint), or as a mental workload (from a more practical, real-world standpoint). Information about mental workload can be particularly relevant in the training of operators of equipment and machinery, such as an operator of a robot manipulator.

However, the existing research has largely approached the issue from a psychological standpoint and not in relation to operator performance. Psycho-regulation levels merely measure a level of activity, such as perception, acquiring new knowledge, or changing one's mind about a particular situation. The parameters measured for mental workload are somewhat different than for psycho-regulation levels. One difficulty in such analysis is the ability to determine how difficult it is for a particular operator to perform a given task. A great deal of research has been performed in order to attempt to measure the difficulty of performing a task for a particular operator. Technologies that have been used in this regard include electro-encephalogram (EEG) measurements, heart rate measurements, skin resistance measurements, or a combination of such approaches.

Eye movement analysis is another approach that has been used for human activity estimation and the measurement of operator performance. Different methods of eye movement measurement have been used for this purpose, such as: electro-oculographic (EOG), electromagnetic, photo-electrical with infra-red source, photo-optical with mirror sensor, video or video-camera means, etc. Such methods analyzed view paths, eye fixation on different external objects, and saccadic movements during changes of visual fixation on objects. This permits an investigation into the main stages of the activity, structure of perceptual processes of information searching, and moments of attention and difficulties, which then link with reasoning processes. These methods allow for estimation of human activity when the human is working with different variants of indicators and interfaces, research and nature of information processes, attention and reasoning.

At present, the main parameter of eye movement being used in view path analysis for the evaluation of human activity is the duration of view fixation. This parameter is considered as a measure of complexity (difficulty) of human activity. It is assumed that the longer the duration of view fixation, the more difficult the activity. In addition, the following parameters are sometimes observed, though not used for the direct evaluation of mental workload of human activity: the time of view fixation of separate external objects; the order and frequency these fixations; amplitude and velocity of saccades when changing the spots of view fixation; values and velocity of drift eye motion in the process of view fixation, etc.

However, using the parameter of duration of eye fixation in order to estimate mental workload of human performance (performance difficulty) has serious deficiencies. This approach focuses mainly on the external side of human performance, which includes relatively simple perceptual and sensory processes related to information searching and perception. But for more complex regulation processes when a human imagines a logic picture, such as making an important responsible decision or thinking about an unusual idea, he may remove himself from the surrounding visual situation (situational awareness) and immerse himself in his internal world. Therefore, the direction of view and a subject's fixation on a particular object are not indicative of mental workload in such a case, though significant mental activity is being undertaken.

So, using the notion of "duration of fixation", which focuses on the external side of activity, in general has serious ambiguities as an indicator of mental workload. Moreover, the term "eye fixation" supposes a stillness of the eye about a certain external object. However, during fixations it is possible to observe slow, involuntary drift eye movements that move a view about and from the object. This has lead to sufficient ambiguities in differences between the separate fixations.

Some other methods have been attempted, such as in U.S. Patent No. 6,090,051 issued to Marshall on July 18, 2000 and entitled "Method and Apparatus for Eye Tracking and Monitoring Pupil Dilation to Evaluate Cognitive Activity". In this case, the parameter of pupil activity is used, instead of the duration of view fixation, as a possible indicator of mental workload, or cognitive activity. However, the parameter of pupil size or activity has since been found to give inaccurate results in terms of trying to measure cognitive activity. This inaccuracy is due to the effects that ambient light and a person's interest in a particular task can have on pupil dilation, each of those being unrelated to cognitive activity with respect to a particular task, thereby potentially producing inaccurate results and/or analysis.

US-A-6 102 870 discloses a method for measuring mental states including thinking and thus mental workload for a human based on rapid eye movements, comprising: identifying saccades in received eye , movement data; computing saccadic parameters; and determining the mental workload based on computed saccadic parameters.

Further research has been done in this area, with an attempt to find a parameter that is related to mental workload but is not as greatly affected by other unrelated factors. The human eye employs not only patterned motion such as observed in scan patterns, but also rapid motion, called saccadic movement. Saccades are eye movements that have only rapid eye movement components, i.e. no slow movement components. In most cases, there is constant saccadic movement of some sort; sometimes these saccadic movements stop, i.e. disappear or are depressed or diminished. Some research has been done with respect to perception, in which saccade speed, movement and orientation were observed, as were "Inter Saccadic Intervals" (ISI). However, such intervals were not studied, but simply observed.

## SUMMARY OF THE INVENTION

It is an object of the present invention to obviate or mitigate at least one disadvantage of previous mental workload estimation approaches.

The present invention provides a method and system that implements approaches by way of which mental workload can be calculated based on a measurement of inter saccadic intervals. A general equation and a more precise equation are provided. The calculation of mental workload according to an embodiment of the present invention was found to be repeatable and valid when tested on many different operators, providing them with tasks of varying difficulty levels. For instance, manipulating an object by one coordinate axis is much easier than manipulating an object by three coordinate axes.

In an aspect, the present invention provides a method of measuring a mental workload for a human based on rapid eye movements, including the following steps: identifying saccades in received eye movement data; computing inter-saccadic intervals for the received eye movement data; and determining the mental workload based on the computed inter-saccadic intervals.

The step of identifying can include an automatic saccade determination scheme, which can be a velocity detection method. The velocity detection method can include the following steps: computing eye movement speed based on the eye movement data; comparing computed speed to a threshold value or level; and determining presence of saccade if computed speed is above the threshold. The step of computing eye movement speed can include: smoothing with a low-pass digital filter to remove noise; and numerically differentiating the filter output.

The step of computing inter-saccadic intervals can include: identifying a boundary of a saccade; computing an inter-saccadic interval between the boundary of the current saccade and a boundary of a next saccade; and storing parameter values in a memory. The step of identifying the boundary of the saccade can include identifying a start of the saccade in response to a measured speed of eye movement being more than a threshold. The step of identifying the boundary of the saccade can include identifying an end of the saccade in response to a measured speed of eye movement being less than a threshold.

The method can further optionally include any of the following steps: receiving the eye movement data; using the determined mental workload value as feedback for training; identifying a peak mental workload value for a period of time; and/or determining a real-time mental workload value.

In another aspect, the present invention provides an apparatus for measuring a mental workload for a human based on rapid eye movements, including: means for identifying saccades in eye movement data; means for computing intersaccadic intervals in the received eye movement data; and an analysis module for determining the mental workload based on the computed inter-saccadic intervals.

The apparatus can further include an ocular sensor for obtaining the eye movement data, which can be at least one video recorder, such as a digital video camera. Alternatively, the electro ocular (EOG) sensor can include: electrodes; an amplifier, and an analog/digital (A/D) converter and a computer processor, such as in a PC.

The apparatus can further include a feedback unit for modifying a training program based on the determined mental workload. The feedback unit can include an evaluation module for evaluating the determined mental workload in relation to stored mental workload data. The feedback unit can also include a control means for controlling access to a device being operated by an operator, based on an output of the evaluation module. The feedback unit can further include a display means for displaying mental workload data based on the determined mental workload.

In a yet further aspect, the present invention provides a computer-readable medium including, or having stored thereon, statements and instructions which, when executed, cause a computer to perform the steps of: identifying saccades in received eye movement data; computing inter-saccadic intervals for the received eye movement data; and determining the mental workload based on the computed inter-saccadic intervals.

In a still further aspect, the present invention provides a signal embodied on a carrier wave, the signal comprising: a first data segment for identifying saccades in received eye movement data; a second data segment for computing inter-saccadic intervals for the received eye movement data; and a third data segment for determining the mental workload based on the computed inter-saccadic intervals.

Embodiments of the present invention can use a determined mental workload measurement as a means of determining a number of useful indications, including but not limited to: human performance based on the measuring of ISI in eye movements; human performance dynamics based on measuring changes in ISI; a total mental workload of human performance; a current mental workload of human performance; a current mental workload of human performance in real-time, e.g. on-line; human proficiency level in simulators and real conditions; interface perfection of a man-machine system; software usability; complexity of human controlled procedures; presenting feedback about the current mental workload of human performance to human in real-time, e.g. on-line; changing a human controlled system mode or training scenarios in response to the current mental workload of human performance; human proficiency properties; level of human mood states in extreme conditions of operator performance; level of human properties and peculiarities in mental processes in any one of education, medicine, sport training, and the like.

Other aspects and features of the present invention will become apparent to those ordinarily skilled in the art upon review of the following description of specific embodiments of the invention in conjunction with the accompanying figures.

## BRIEF DESCRIPTION OF THE DRAWINGS

Embodiments of the present invention will now be described, by way of example only, with reference to the attached Figures, wherein:

Fig. 1 is a flowchart illustrating a method of measuring mental workload according to an embodiment of the present invention;
Fig. 2 is a flowchart illustrating steps involved in the step of identifying saccades of Fig. 1;
Fig. 3 is a flowchart illustrating steps involved in the step of computing inter-saccadic intervals of Fig. 1;
Fig. 4 is a graphical illustration of ISI duration for human activity;
Fig. 5 is a graphical illustration of ISI taxons for human activity;
Fig. 6 is a block diagram of a system according to an embodiment of the present invention;
Fig. 7 is a diagram of a system according to another embodiment of the present invention;
Fig. 8 is a block diagram of a system incorporating feedback according to an embodiment of the present invention; and
Fig. 9 is a screen shot of a display showing ISI values for a particular human activity for use as feedback for a training system.

## DETAILED DESCRIPTION

Generally, the present invention provides a method and system for operator mental workload measurement and analysis, which measures time intervals between saccades in eye movements. These intervals, called Inter Saccadic Intervals (ISI), characterize different levels of mental workload during operator performance, such as for MRO (Operator of Robot Manipulator). The device can include electro-oculogramic (EOG) sensors for measuring oculometric potentials from the human face, an electronic biosignal amplifier for amplifying EOG potentials, an analog-to-digital (A/D) converter for generating digital output signals relating to the EOG potentials, and a digital computer for continually measuring the electrooculogramic potential, extracting the saccades, calculating ISIs, and then determining quantitative criteria of mental workload based on the ISIs. Human performance can be measured and analyzed: for a proficiency level evaluation in simulators and real conditions; for estimation of automated control system's interface and software usability; as well as for human controlled procedures complexity evaluation.

<u>Definitions</u>

The term "mental workload" as used herein represents a measure of the complexity or difficulty of performing a particular task. Mental workload can be measured for individual operators, and typical ranges of mental workload can be developed for the performance of certain tasks. Those ranges, as well as previous operator-specific measurements, can be used as feedback for training purposes. According to embodiments of the present invention, it is possible to measure a total mental workload for a complete duration of task performance, or to measure a mental workload for a portion of the duration, or even at a particular instance. Although the term "mental workload" is used primarily in relation to a "humans" or to "human performance" in the description, the terms "human" and "human performance" are to be understood to include humans and other primates for whom it is known in the art that eye behaviour is substantially similar. Of course, if such measurements are used for non-humans primates, e.g. for monkeys, one skilled in the art would readily understand that discussions relating to thresholds and taxon levels for humans would be suitably modified to reflect functionally equivalent levels and/or thresholds for the subject being considered.

The term "inter-saccadic interval" or "ISI" represents a time interval between saccades in eye movements. An ISI can be determined by computing an inter-saccadic interval between a boundary of a current saccade and a boundary of a next saccade. The boundary can be the start of the saccade or the end of the saccade. Therefore, in one embodiment, ISI can be determined by computing an inter-saccadic interval between the start of the saccade and the start of a previous saccade. This obviously means that the duration of the saccade, which is minimal, is being included in the measurement. Alternative implementations could measure the difference between the ends of two saccades, or could subtract the duration of the saccade itself from the measurement (i.e. measure the interval between the end of a saccade and the beginning of a next saccade).

__Concepts that form a basis for the invention__

Saccades are sudden and rapid ballistic movements lasting about 10 to 120 milliseconds and traversing from less than 1 to 40 degrees with a peak velocity of about some hundred degrees per second. Saccades are eye movements that have only rapid eye movement components, i.e. no slow movement components. Vision is suppressed during a saccade. Saccadic jumps may be voluntary or involuntary, both of which move the eyes of the viewer between fixations or return them on the fixation if eyes drift from it.

For sensory-perceptual processes, the fixation between two saccades is a comparatively brief period of relative stability which allows a portion of a scene within the foveal vision to be studied. In this case the fixation period is typically from 100 to 600 milliseconds. Small jittery motions occur during a fixation; these motions are composed of slight drifts with amplitudes less than one degree, followed by microsaccadic corrections. Here, a low amplitude, high frequency tremor is superimposed. The purpose of a saccade is to rotate the eye so that the image of a cue in the visual scene falls within the foveal vision. The image is then studied during the fixation. The microsaccadic movements induce changes in the image required for seeing.

For more complex regulation processes when a human imagines a logic picture, makes an important responsible decision or thinks about an unusual idea, he may remove himself from the surrounding situation (situational awareness) and immerse himself in his own inner world. In these cases, human eyes may slowly move in some winding path with saccade depression; intervals (ISIs) between saccades may be very long, such as 30 seconds and even longer.

Finally, blinks start slightly before or simultaneously with upward directed saccades, but never after the eye movement. Blinks are sometimes initiated after the onset of downward directed saccades. It has been reported that only in less than one percent of blinks is there apparent eye movement. Blinks usually last from 20 to 200 milliseconds with the longest observed terminating smoothly around 300 milliseconds.

The ISI parameter is free from many of the deficiencies of previous parameters or factors used in mental workload, or human activity, analysis. It is related to both external and internal aspects of human performance, and does not require view stillness. Saccades also occur when eyes are closed. At the same time, it is known that ISI duration is related to human activity difficulty. So this parameter is more appropriate as a parameter used for measurement of mental workload of human performance than other parameters used in the prior art. In previous experiments relating to ISI, different levels were assigned to different complexities of tasks as they relate to the duration of ISI. A task involving only simple motor skills would be at a lower psycho-regulation, or mental workload, level than a task involving detailed mental analysis and determinations. Such regulation or workload levels can be identified as in **Table 1** below. The concept of an ISI taxon will be discussed later.

**Table 1**

| Level No. | Description of Regulation/Workload Level | ISI taxon (sec.) |
|---|---|---|
| 1 | Direct interaction | 0.03 - 1.0 |
| 2 | Mediated co-ordination | 0.9 - 2.0 |
| 3 | Program-purposed organization | 1.9 - 5.0 |
| 4 | Personality-normative changing | 4.6 - 11.5 |
| 5 | World outlook corrections | more than 10.7 |

For instance, suppose a person is driving a car and prepares to stop at a red light, but another car is coming up behind and does not appear to be slowing down. At that instant in time, the person is faced with a situation that is out of the ordinary, and has to decide whether to break the traffic rules and avoid being hit from the rear by the approaching car, or obey the traffic rules and risk being hit from the rear by the approaching car. In such a case, the ISI can be observed in the range of 10-15 seconds. In an analogous manner, if a spacecraft operator encounters a situation wherein he questions whether the instrumentation is working properly, the operator has to determine whether he should depend on his knowledge and experience and go against the instrumentation readings, or whether he should go against his instincts and trust the instrumentation.

With this previous research in mind, it is therefore desirable to be able to provide a system and method for quantitatively measuring the mental workload based on ISI.

<u>**A method according to an embodiment of the present invention**</u>

Embodiments of the present invention can be implemented in software, or in hardware, and typically in a combination thereof. A method according to an embodiment of the present invention will now be discussed. However, it is to be understood that this method can be performed by software according to an embodiment of the present invention, or stored on a computer-readable medium or embodied in a carrier signal which, when executed, performs the steps of the method.

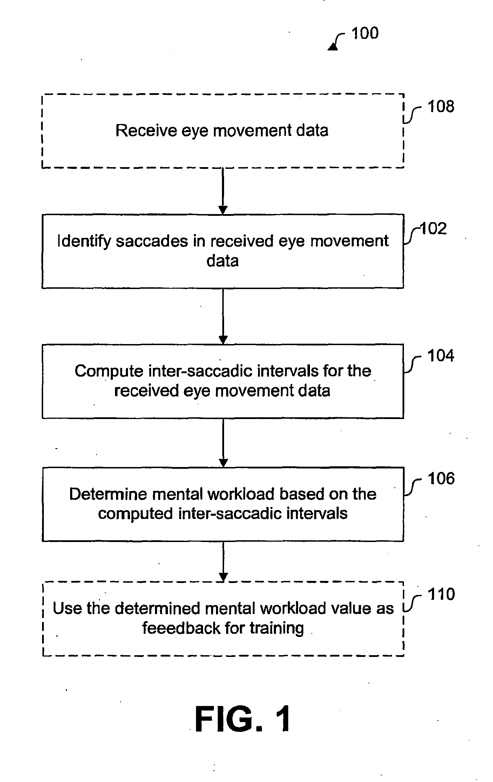

**Fig. 1** is a flowchart illustrating a method of measuring mental workload according to an embodiment of the present invention, in particular a method **100** of measuring a mental workload for a human based on rapid eye movements (saccades). Step **102** includes identifying saccades in received eye movement data. Step **104** includes computing inter-saccadic intervals for the received eye movement data. Step **106** includes determining the mental workload based on the computed inter-saccadic intervals. The method can include the optional step **108** of receiving the eye movement data. The method can further include the optional step **110** of using the determined mental workload value as feedback for training. Of course, each of these steps can include one or more sub-steps.

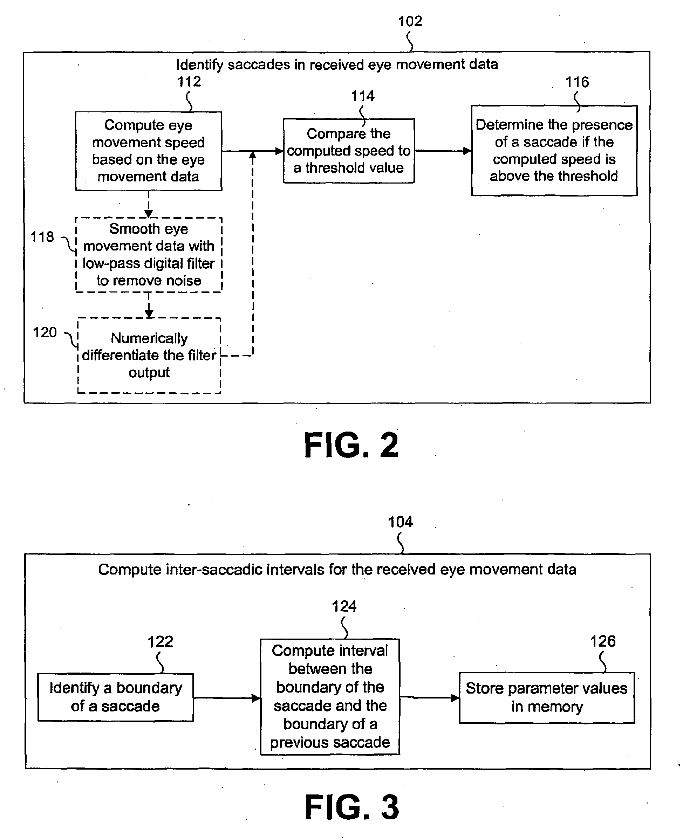

**Fig. 2** is a flowchart illustrating steps that can be involved in the step of identifying saccades of **Fig. 1.** The step **102** can include an automatic saccade determination scheme, such as a velocity detection method. In the case of a velocity detection method, the following steps can be sub-steps of step **102.** Step **112** includes computing eye movement speed based on the eye movement data. Step **114** includes comparing the computed speed to a threshold value. Step **116** includes determining the presence of a saccade if the computed speed is above the threshold. Essentially, these steps include measuring a speed of the eye movement, comparing the measured speed to a stored threshold value, and if the measured speed exceeds the threshold, a start of a saccade is identified, whereas if the measured speed is lower than the threshold, an end of a saccade is identified.

The step **112** of computing eye movement speed can itself include sub-steps, identified as steps **118** and **120.** Step **118** includes smoothing the eye movement data with a low-pass digital filter to remove noise. Step **120** includes numerically differentiating the filter output from step **118.**

In a particular embodiment, the determination scheme that is used can be a velocity detection method where the eye movement speed is computed from the EOG data. Preferably, as described previously in relation to **Fig. 2,** this includes first smoothing with a low-pass digital filter to remove noise and then numerically differentiating the filter output. This helps to filter out frequencies in which saccadic movement is not observed. The computed speed is compared to an empirically determined threshold value, separating saccadic movements from fixations and drifts. An example of such a threshold level is 5 degrees per second. If the computed speed is above the threshold, it is determined as saccadic, whereas if it is less than the threshold, it is considered as fixation or drift. For blink detection, a special symmetric scheme of two saccades analysis is used.

Moreover, for user-specific transition EOG data in eye movement amplitude, a calibration step can preferably precede the data measurement. During the calibration, the human subject moves their view a few times between two points on a computer display, preferably without head movement and blinking. The distance between these points can then be established, in angular degrees and time of point fixation (about 1-2 seconds), thereby completing the calibration.

**Fig. 3** is a flowchart illustrating steps involved in the step of computing inter-saccadic intervals of **Fig. 1.** Step **122** includes identifying a boundary of a saccade, and typically follows the completion of step **116** from **Fig. 2,** though alternative methods are also possible. The method can include an identification of the specific point in time at which the computed speed exceeded the threshold, thereby identifying the start of the saccade. Step **124** includes computing an inter-saccadic interval between the boundary of the saccade and the boundary of a previous saccade. For example, the ISI can be computed between the start of a saccade and the start of a previous saccade. Step **126** includes storing the computed parameter values in memory. The parameter values can include the computed interval and the start time(s) of the saccade(s).

Of course, step **124** can be implemented in alternative fashions. In the example described above, the inter-saccadic interval is computed by measuring the difference between the beginning of a saccade and the beginning of the next (or previous) saccade. This obviously means that the duration of the saccade is being included in the measurement. However, the duration of the saccade is minimal and is not significant when compared to the interval between the saccades. Therefore, any minor contribution of the saccade duration to the measurement does not have a material effect on the accuracy of the measurement. Alternative implementations could measure the difference between the ends of two saccades, or could subtract the duration of the saccade itself from the measurement. A start of a saccade can be identified in response to a measured speed of eye movement being more than a threshold. An end of the saccade can be identified in response to a measured speed of eye movement being less than a threshold.

Using a computer program, which implements the background knowledge described above, a processor can execute a method or routine that carries out an automatic saccade determination scheme. The routine detects saccades, as well as separate saccades from fixations, drifts and eye blinks.

Software that can be used with a system and method according to an embodiment of the present invention is designed for singling out saccades and evaluating ISIs in real time as well as displaying, in the form of a diagram, duration of ISIs. It is also possible to directly record EOG signals into a file, which allows their visualization and interactive analysis. To determine sensitivity of the measurements, a calibration regime can be provided, which operates based on two or more marks given on the display.

The method uses an algorithm for singling out saccades on the basis of velocity, with the threshold of singling out saccades set at about 5 deg./sec., which is in accordance with recommendations in the following references: Yu. Berger, A. Luuk and T. Mogom, "A comparative analysis of algorithms for detection of saccades", Automated Real Time Systems for Ergonomic Studies, Tartu, 1988, pp. 271-274; and Yu. Berger and T. Mogom, "The effect of Ways of Processing a Signal upon Parameters of Saccadic Eye movement", Automated Real Time Systems for Ergonomic Studies, Tartu, 1988, pp. 274-277. Besides, in the process of analysis of an EOG signal, eyelid blinking is excluded.

To determine ranges of ISI changes at each level of control, modifications of one of the methods of taxonomic analysis can be used, e.g. a method for singling out formal elements termed "FOREL-1" and disclosed in N.G. Zagorujko "Methods of Identification and Their Use", Moscow, Sov. Radio. 1972.

The method employed according to an embodiment of the present invention preferably belongs to the class of iterational procedures, which permits processing of large arrays of data. Taxons here have the form of degenerated hyperspheres of an individual size (scale) which are characterized by a radius and centre of gravity (or just centre) obtained on the basis of averaging ISIs included by the hypersphere. ISIs situated at a distance from the centre which is less than the radius of the hypersphere, are considered as belonging to the hypersphere.

Parameters of taxons for various levels of mental processes have been presented in **Table 1.** The uncertainty of taxons is manifested by their overlapping, i.e. the lower border of each subsequent taxon is less than the upper border of the preceding one. Thus ISis which are within the overlapping range, cannot be exactly associated with a specific level of mental processes. Uncertainty of borders between adjoining taxons increases as these levels become higher.

For a quantitative evaluation of performance in the process of experimental studies, parameters of ISI taxons are preferably introduced into the method of processing of EOG signals. This evaluation was performed by relating each recorded ISI to a certain taxon by comparing it to values of upper borders of taxons. The presence of taxons makes a quantitative evaluation of ISIs (on the basis of numbers of taxons) a qualitative one (based upon levels of control).

In an aspect, the present invention provides a computer-readable medium including, or having stored thereon, statements and instructions which, when executed, cause a computer to perform the steps of: identifying saccades in received eye movement data; computing inter-saccadic intervals for the received eye movement data; and determining the mental workload based on the computed inter-saccadic intervals.

In another aspect, the present invention provides a signal embodied on a carrier wave, the signal comprising: a first data segment for identifying saccades in received eye movement data; a second data segment for computing inter-saccadic intervals for the received eye movement data; and a third data segment for determining the mental workload based on the computed inter-saccadic intervals.

Of course, in the embodiments involving a computer-readable medium and signal embodied on a carrier wave, statements and instructions and/or data segments can be provided to enable the performance of other optional method steps as described herein.

**Determining Mental Workload**

With respect to the steps of determining, or computing or calculating, the mental workload based on received eye movement data, such as ISI data, two primary ways of performing this determination will be described below. Of course, it is to be understood that other manners of determining the mental workload based on received eye movement data may be used, as would be obvious to one of ordinary skill in the art.

A first determining method is more involved and includes some equations. However, this method can yield more accurate results. This first determining method uses the "taxon" tools as described above, and can be split into five core routines, or methods, as well as a sixth routine that varies based on the end result desired:

Routine 1 computes the current mental workload of human performance (activity):

$$W_i = \frac{B_i}{B_1} * t_i \qquad \text{Equation 1}$$

where: $W_i$ represents the current mental workload of human activity (in seconds) for current ISI; i represents the number of current ISI; $B_i$ represents the maximum border of taxon (in seconds), which belongs to current ISI; and $t_i$ represents the duration of current ISI (in seconds).

Routine 2 then computes the value of the current mental workload of human performance on-line for presentation on a display means, such as a computer monitor, as feedback to the human subject. Routine 3 then computes the value of the current mental workload of human performance on-line for changing of the training scenario (less or more difficult) or human controlled system modes when it reaches its threshold quantity.

Routine 4 checks a program finish command from keyboard. If the result is true, Routine 5 computes the total mental workload human performance criteria - total mental workload.

These criteria are an estimation of human performance from the total data of time intervals of different regulation processes levels. The main problem in such estimation is a comparison of different levels of ISI having differences in quality. But the quality of some regulation process levels changes when its taxon maximum border is exceeded. Therefore the quality of different levels of estimation may be estimated as the relation of each taxon maximum border to the maximum border of the first taxon. Then the human activity criteria are presented in the following **Equations 2** and **3.**

$$Ws = \sum_{i=1}^{N} W_i \qquad \text{Equation 2}$$

where: $W_s$ represents the total mental workload of human activity (in seconds); $i$ represents the number of ISI; $W_i$ represents the current mental workload of human activity (in seconds); $N$ represents the total number of ISI in performance; and $t_i$ represents the duration of $i$ ISI (in seconds).

$$Wa = \frac{Ws}{N} \qquad \text{Equation 3}$$

where: $W_a$ represents the average of mental workload of human activity; $W_s$ represents the total mental workload of human activity (in seconds); and $N$ represents the total number of ISIs.

A second determining method, in contrast to the first determining method described above, calculates the total duration of all inter saccadic intervals and then divide the total by the number of interval units. This simple method is applicable to measuring mental workload for the entire task performance, as well as for measuring mental workload for a part, or a portion, of task performance. However, this second determining method doesn't take into account the changing quality of workload in transitions between different levels. In this case workload has linear interpolation, in other words ISI taxonomy is not used:

$$W_i = t_i \qquad \text{Equation 4}$$

where: $W_i$ represents current mental workload of human activity (in seconds) for current ISI; i represents a number of current ISI; $t_i$ represents the duration of current ISI (in seconds). When Equation 4 is used, the same **Equations 2** and **3** can be used to determine $W_s$, the total mental workload of human activity, and to determine $W_a$, the average of mental workload of human activity.

Routine 6 can compute (or measure, or determine) any one of a number of indicators or parameters based on the result of Routine 5. Embodiments of the present invention can use a mental workload measurement (determined in any manner as described above) as a means of determining a number of useful indications. For instance, it can measure the human proficiency level in simulators and real conditions by comparing the total mental workload of human activity with its normative (baseline) values. It can alternatively compute the interface perfection of the man-machine system by comparing of the total mental workload, and mental workload of human activity for different variants of interface. Other possible parameters that can be measured include, but are not limited to: human performance based on the measuring of ISI in eye movements; human performance dynamics based on measuring changes in ISI; a total mental workload of human performance; a total and current mental workload of human performance; a current mental workload of human performance in real-time, e.g. on-line; software usability; complexity of human controlled procedures; presenting feedback about the current mental workload of human performance to human in real-time, e.g. on-line; changing a human controlled system mode or training scenarios in response to the current mental workload of human performance; human proficiency properties; level of human mood states in extreme conditions of operator performance; level of human properties and peculiarities in mental, cognitive and regulative processes in any one of education, medicine, sport training, and the like. Of course, each of these routines or computations are optional and are only performed when necessary or when requested.

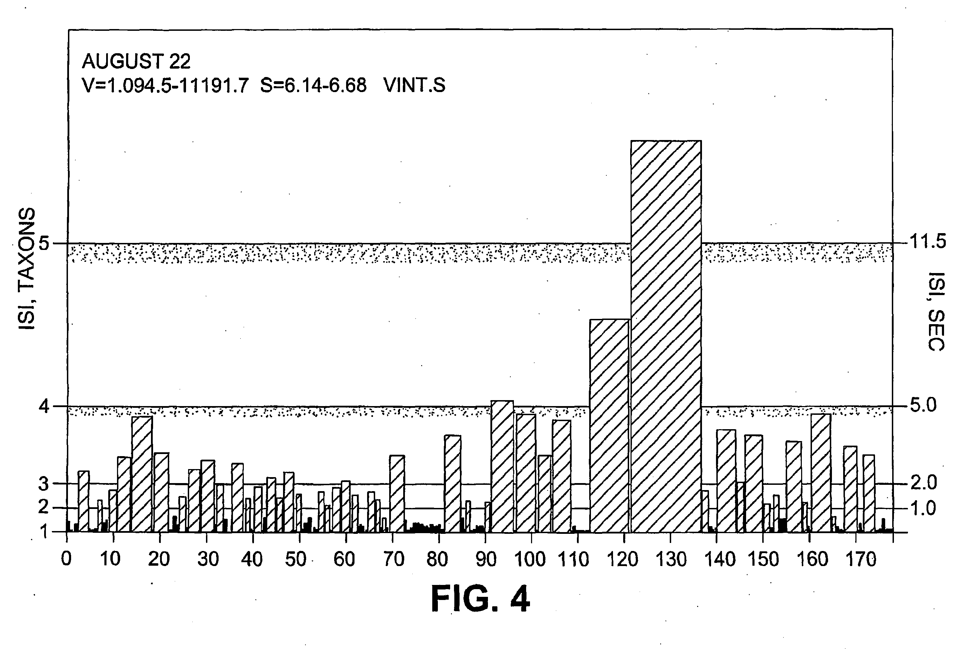

Observing the mental workload of human performance over different time intervals allows the method or system to measure the dynamics of human performance difficulty based on the parameter of ISI duration, which can be represented visually. **Fig. 4** is a graphical illustration of ISI duration for human activity. Referring to **Fig. 4,** in the diagram of ISI duration the abscissa axis presents the activity time (in seconds), the right ordinate axis - the ISI duration (also in seconds) and the left one - the taxon's numbers. Fuzziness between taxon borders, such as for taxons 4 and 5, is shown in **Fig. 4** as horizontal regions including dots. Another routine can compute this diagram in real time and show it on a display, such as a computer monitor.

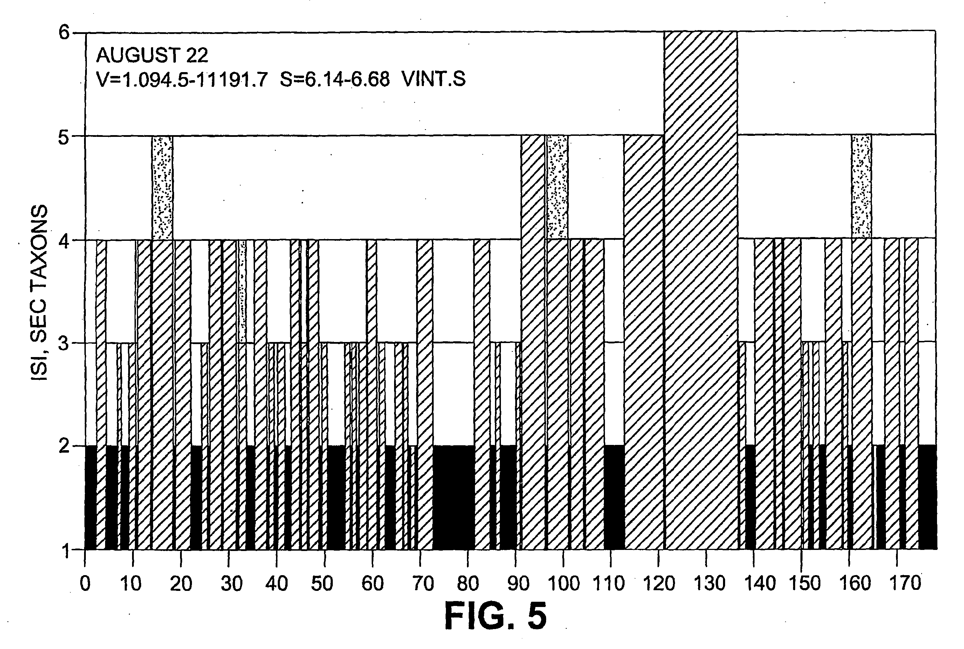

**Fig. 5** is a graphical illustration of ISI taxons for human activity. Relative types of activity dynamics are presented in the diagram of ISI taxons, which can also be computed by the Routine 6. Referring to **Fig. 5,** in this diagram the abscissa axis presents also the activity time (in seconds), and the left ordinate axis presents the taxon's numbers. Fuzziness between taxons borders are shown at the **Fig. 5** as column portions having dots (i.e. portions between taxon numbers 4 and 5).

Thus the method can obtain, in real time, a graphical representation of dynamics of performance in the form of two diagrams relating to ISI durations and their taxons. In both cases, duration of performance (seconds) is plotted along the horizontal axis. In the first case, such as in **Fig. 4,** ISI durations are plotted along the vertical axis on the right scale (also in seconds) along with upper borders of taxons on the left scale (they are represented by a dotted line). In the second case, such as in **Fig. 5,** the number of taxons is plotted along the vertical axis.

The routine 6 can compute the value of the current mental workload of human performance on-line for presentation on computer monitor as the feedback to human. The routine 6 can also compute the value of the current human activity complexity on-line for changing of the human controlled system modes or training scenarios when it reaches its threshold quantity.

Optionally, the routine 6 checks a program finish command from a keyboard. If the program finish value true, the routine computes the total mental workload of human performance criteria. These criteria can be considered a measurement, or estimation, of human performance from the total data of time's intervals.

The routine can compute the human proficiency level in simulators and real conditions by comparing of the mental workload of human performance with its normative values.

<u>General system implementation</u>

In another aspect, the present invention provides an apparatus for measuring a mental workload for a human based on rapid eye movements, including: means for identifying saccades in eye movement data; means for computing inter-saccadic intervals in the received eye movement data; and an analysis module for determining the mental workload based on the computed intersaccadic intervals.

A system incorporating embodiments of the present invention can be implemented by means of hardware, software, or a combination thereof. The particular embodiments described herein will be a combination of hardware and software components. However, it should be understood that many of the software components could be implemented in hardware, and vice-versa. Although embodiments of the present invention relate to the analysis and interpretation of eye movement data, some embodiments described herein also include optional components relating to the acquisition of eye movement data.

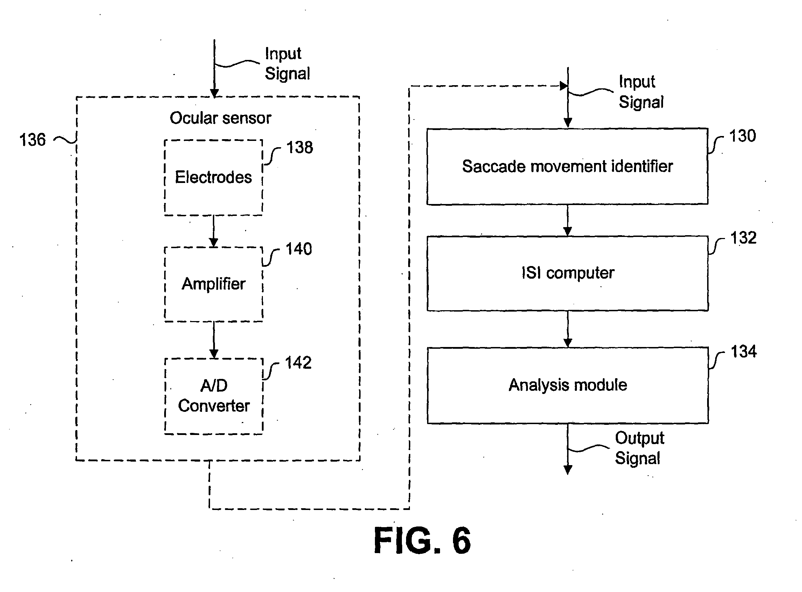

**Fig. 6** is a block diagram of a system according to an embodiment of the present invention. The system includes a saccade movement identifier **130,** or means for identifying saccades in eye movement data. An ISI computer **132,** or means for computing inter-saccadic intervals, is in communication with the saccade movement identifier **130** for computing inter-saccadic intervals in the received eye movement data. An analysis module **134** is provided for determining the mental workload based on the computed inter-saccadic intervals.

In the analysis module **134,** it is possible to calculate the total task workload, as well as to calculate workload levels for different time periods in the task performance. The peak of performance difficulty can also be identified based on the measured value. The output signal can be used as feedback, which can then indicate what type of training may be needed based on the measured values. In such a case, the apparatus can include a feedback unit for modifying a training program based on the determined mental workload. The digital output signal of the analysis module represents parameters of time intervals with different levels of regulation processes in human activity.

With respect to eye movement measurement, there are many ways in which eye movements have been measured in the past, such as scan pattern measurement and even saccadic movements using different hardware. Such hardware includes a helmet having laser or other light sources, which are not very suitable since they require the subject to remain very still and therefore will likely modify the subject performance and reaction in the operation of certain tasks. Though such arrangements provide very accurate measurements, the measurements may not be extremely relevant due to the potential modification of the operator's behaviour, and it is very intrusive to the operator to use in the course of normal operation. Moreover, such systems and apparatus are very expensive.

According to embodiments of the present invention, the apparatus can further include an ocular sensor **136** for obtaining the eye movement data, which can be a video ocular sensor, such as a digital video camera, or an electro ocular sensor. The electro ocular (EOG) sensor can include: electrodes **138** for recording potentials; an amplifier **140,** such as an amplifier block with a galvanic junction; and an analog/digital (A/D) converter **142,** which can also power the amplifier block. An IBM PC-type personal computer (not shown) can advantageously be provided, into which the output of the A/D converter is provided. A particular implementation uses an alternating current amplifier with a time constant not less that 10 seconds, which is in accord with recommendations for amplifiers of this type for reliable singling out in the signal measured of fast eye movements which are presented, for example, in A.M. Boukadoum and P.Y. Ktonas "EOG-based recording and automated detection of sleep rapid eye movements: a critical review, and some recommendations", Psychophysiology, Vol. 23, No. 5,1986, pp. 598-611. Besides, this equipment meets requirements of medical hardware as far as electrical safety is concerned, and is licensed (authorized) for use in psychophysiological research.

Providing an ocular sensor **136** as a video ocular sensor is an example of a non-contact method of measuring eye movements. One example uses digital camera technology (video-oculography - VOG). In this example, two digital cameras are preferably set up away from the operator but facing the operator so as to capture eye movements in the digital cameras. A first camera is pointed at one of the operator's eyes. A second camera is pointed at the operator's face. Using high resolution digital cameras, a very accurate measurement of eye movement can be obtained. Furthermore, this non-contact measurement has little or no effect on the operator's behaviour and performance, and is not intrusive in the course of normal operation.

For the measurement of eye movements, any of the following elements can be used: video or cine-camera means; photo-electrical means; photo-optical means with mirror sensor; electromagnetic sensor means. Digital computer means coupled with the sensor means can be used for computing the saccadic eye movement data. The ocular sensor can include means of measuring the value of ISI of eye movements using signals from a human face.

Consider an exemplary implementation of an embodiment of the present invention in a practical measurement of mental workload. In this example, operators were provided with an object that the operator has to reconfigure. The operator is provided with pitch, level, degree, roll, and other parameters relating to the object to be manipulated. The object to be manipulated was a scale model to be manipulated. The operator performs a task over a period, such as one minute. During each time sub-interval, the operator has variations in the amount of mental workload, which variations can be observed in **Fig. 3** and **Fig. 4,** as described earlier. Many previous approaches simply provided an average measurement of mental workload over the period of time of the task performance.

According to embodiments of the present invention, it is possible to isolate particular time periods in which the Inter Saccadic Interval was very high, and therefore the operator required a much higher mental workload in order to achieve the task. For example, when manipulating an object in space, the axes of movement may not be the same as the frame of reference in which the operator is located. For instance, in situations where an operator must constantly evaluate the intensity and distance by which a hand controller must be displaced in order to move an object, this will require a great deal of mental workload, possibly over the entire time of task completion. A task such as this one would be a manual inspection of an object, in which the inspection involves tracking an arm in a circular direction, which is very difficult as compared to simply performing a visual inspection. Also, when performing circular motions, the operator must be aware of changing negative and positive values in the X and Y directions in order to achieve a circular motion. During experimental observation, it was observed that a great Inter Saccadic Interval existed at the boundaries where the operator had to consciously change from movements in a positive and negative direction in a particular axis.

In contrast to the prior art, embodiments of the present invention permit the identification and evaluation of a peak value of mental workload during the time of task performance. Using this ability to identify peak times of difficulty, it is possible to take the results from different operators having different skill levels, and determine time periods where each of the operators would experience a heightened difficulty in performing the task, regardless of their varying experience and ability. This provides the ability to empirically prove certain assumptions, such as the assumption that it is more difficult to manipulate objects when the operator is closer to the object than when the operator is farther away from the object. An example of this situation is that movement of an end-effector of a robot-manipulator when the operator is closer to the other interactive object structure (space station or Space Shuttle elements) is easier than when the operator is farther away from the interactive object structure. (e.g. free space positioning of the robot manipulator).

In another example that was observed according to embodiments of the present invention, operators were placed in a situation where a particular apparatus was operating in an automatic mode, such as an autopilot mode. In such a case, the operator typically has a lower mental workload since the operator is simply observing and making sure that the automatic system is functioning properly. The operator in this case is acting as a backup to the automatic system. In the experiments, an anomaly was introduced so that the automatic system did not perform properly. The operator's reaction and mental workload were then observed. The anomalies can be accomplished by means of showing a malfunction in the system.

Measurement system implementation

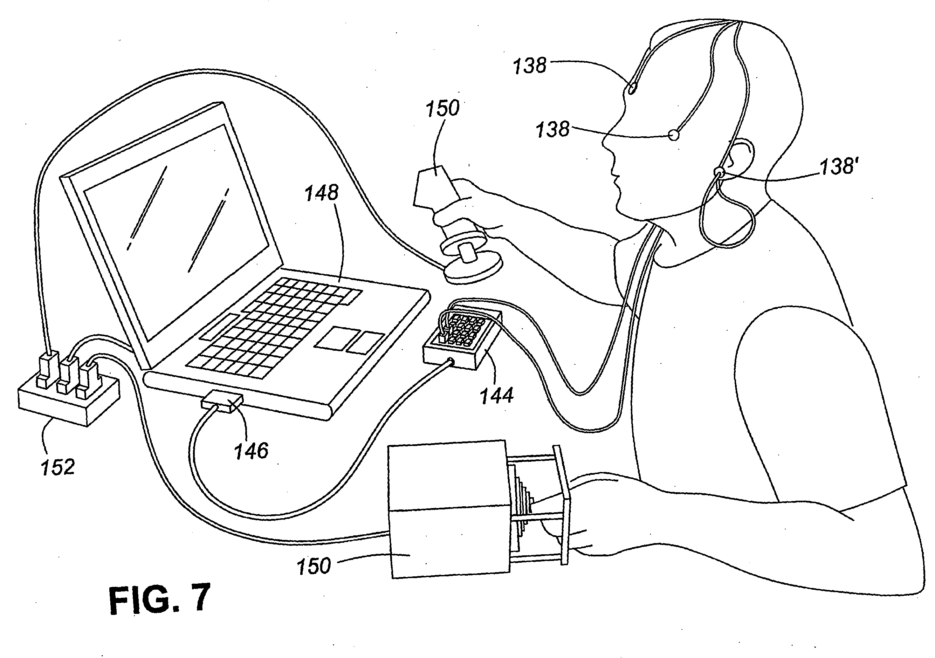

In specific embodiments of the present invention, a simple and inexpensive eye movement measuring device is used, namely an electro-oculogram. The EOG offers a precise enough measurement and is inexpensive to implement. **Fig. 7** is a diagram of a system according to another embodiment of the present invention, such as a system employing EOG. In typical use, sensors or electrodes **138** are placed on the face around the operator's eyes in order to measure the electrical voltage changing from eye rotating so as to measure the ISI. Typically, a placement of four sensors or electrodes in a mask configuration around the operator's eyes has been used. It has also been found that it is possible to include only three sensors **138,** as shown in **Fig. 7,** placed in a triangular arrangement around the mask of the eyes in order to obtain accurate measurements of ISI without requiring four sensors. Each of these arrangements makes use of the fact that eye movement creates voltage changing near the eyes. The three sensor configuration was found to provide more reliable results, since the four sensor configuration often picked up muscle movements related to other facial movements and unrelated to eye movements, thereby introducing extraneous data that could throw off the measurements.

A periocular electrode array using four electrodes placed about the eyes of the viewer can be employed according to an embodiment of the present invention. Two electrodes can be located horizontally at opposing temple sites and two more electrodes located vertically at opposing sites above the eyebrow and below an eye of the viewer. The Electro-oculogram is generated by the retinal to corneal electrostatic potential of the eye.

The three sensor configuration uses trigonometric measurements in order to measure ISI in an operator. These measurements make use of the fact that the human eye is essentially a electrical dipole. When a dipole (e.g. the eye) rotates , it creates a change of an electric field and a difference of potentials between two electrodes. Although the resulting signal is at a very low level, the electrodes being used can acquire such signals. After the electrodes **138** have acquired this electric potential measurement, the signal is passed on to the amplifier **140** (not shown in **Fig. 7**), which can include a plurality of amplifiers. The amplifier amplifies the signal in a sufficient manner to be manipulated by the system software. From the amplifier **140,** the signal is optionally transmitted to an interface box **144.** The interface box preferably protects the human subject from the possibility of electrical shock. The interface box can include its own power supply, such as batteries. The interface box's power supply can be used to provide power to the amplifiers. An example of a suitable interface box is a 16 channel biovision™ data acquisition box. With such a data acquisition box, only two of the channels would typically be used in accordance with embodiments of the present invention. However, the other channels could be used to measure other parameters or factors at the same time.

Subsequent to entering the interface box **144,** the data is transmitted to a data acquisition card **146.** The data acquisition card can include an analog to digital converter **142** (not shown in **Fig. 7**). The data acquisition card can be any data acquisition card, such as the DAQ Card™ 6024E from National Instruments. When the electrodes are being used, typically a ground connection is provided, shown as **138'** in **Fig. 7,** which can be placed on the ear or on the neck or in any other suitable location. The electrodes can include a central electrode which is preferably placed low on the subject's forehead above the top of the bridge of the nose and above the space between the subject's eyebrows. Side electrodes are preferably placed slightly underneath and behind each of the subject's eyes, preferably on the top of the cheekbone. Various filters and other adjustments can be used in the set up of the system. These filters are used so that the electrodes measure only rapid motion in order to acquire the ISI parameters desired according to embodiments of the present invention.

Whereas prior art arrangements using four contacts use an orthogonal frame of reference, the three contact arrangement of preferred embodiments of the present invention use a trigonometric frame of reference with trigonometric functions to interpret the measurements.

When the signal is received into the analog/digital converter, it is obviously converted to a digital signal and passed on to the system software for analysis. The data is provided in real time to an analysis module. The analysis module can be similar to that disclosed in the previously filed and commonly assigned U.S. Patent Application Serial No. 10/455,709. In such a situation, it is possible to compare measurement of hand controller displacement, and end effector of robot manipulator affecter movement, as well as Inter Saccadic Intervals. The analysis module can be used to analyze real time data. Alternatively, the analysis module can also be used to analyze data that has been previously acquired or captured. The analysis module can be implemented separately while acquiring measured data from a database, or can be integrated into a simulation module or training module. The analysis module can be implemented in any variety of software implementation, such as using the commonly known MATLAB™ simulation tool.

The skin surface electrode configuration for the measurement of the vertical and horizontal Electro-oculogramic signals can be made from conductive cloth electrode sensors. The electrode-sensors can be made from woven, stretchable, nylon fabric impregnated with silver particles, and a porous fabric can support an electroconductive gel placed between the skin and electrode material. As with all skin-surface electrode placements, the user preferably applies a skin preparation to the skin surface to remove skin oil and outer dead skin cells before fitting the electrode support fixture, to reduce skin resistance to an acceptable level (below 5K ohms).

The amplifier **140** can contain dual input differential instrumentation preamplifiers referenced to an indifference electrode, for amplifying the microvolt signals measured by the electrode configurations. The preamplifiers are input buffered for high input impedance with high gain, low noise, and appropriate isolation to safeguard the user from electrical shock. The electrode lead wires can be twisted and the components of the amplifier can be housed in a shielded enclosure to reduce interference from outside sources of electromagnetic energy. Because of the microvolt signals, a summing amplifier is preferably used to reduce the common-mode response to the direct current (DC) offset potential induced at the interface between the electrodes and skin surface. This potential is stable for proper electrode preparation and application. As is common practice for low signal amplifier circuits, the output of the summer can be further amplified by a second stage programmable gain amplifier. After the second stage amplification, the signal is preferably high-pass filtered (with about 0.1 Hz cut-off) to remove electrode offset potential and low-passed filtered to remove externally induced noise. For user's safety, the amplifier stages can be powered from A/D converter **142** at a digital computer **148,** and their outputs can be transmitted through optocouplers to provide isolation from other power supplies.

In practice, the DC offsets generated by the corneal to retinal potentials will change with light adaptation, and thereby drifting over time out of range of the power supplies results in clamped amplifiers. For these reasons and because it is necessary rapid eye movements (saccades) measurement, the amplifier **140** can be implemented as an alternating current (AC) differential instrumentation amplifier with time constant not less 10 seconds which is continually updated the AC bias correction voltage. In practical implementation, the amplifier **140** can be provided in the wires connecting the electrodes **138** to the interface box **144,** or can be provided in the interface box itself.

Once the amplified skin surface electrode voltage measurements from the instrumentation amplifier **140** are obtained, these measurements are input as analog inputs to the multiplexed analog-to-digital (A/D) converter **142** where the signals are digitized for further processing. The digitized signal outputs from the A/D converter **142** are input to the digital computer **148** which, in turn, performs several digital operations on the input signals.

In practical implementation, a digital computer routine reads the EOG data from the A/D converter **142** output into a memory buffer ordered by time-code. Another digital computer routine categorizes the eye sight state as saccade or fixation, eye blink or slow movement (drift) from the received EOG data. These routines can be instructions embodied in a carrier signal or wave, or stored on a computer readable medium, which, when executed, perform the steps in the routines.

Optional elements are also shown in **Fig. 7.** The system can include one or more hand controllers **150**, as well as a hand controller interface box **152**. The hand controller interface box is for receiving signals and data from the hand controller(s) **150**, and can include an A/D converter. The hand controller interface box **152** is in communication with the computer **148** for providing hand controller information to be processed/analysed, and can supply power to the hand controller(s).

Of course, the number and definition of human performance regulation levels used in conjunction with embodiments of the present invention are simply exemplary. Any other definition of human performance regulation levels can be used, and the method and system can easily be modified accordingly, as is obvious to one of ordinary skill in the art. Also, other criteria can be derived from the data of ISI, such as data based on the current and total mental workload of human activity.

<u>System Implementation including feedback</u>

Embodiments of the present invention also seek to use the parameter of mental workload in order to assist in operator training. Feedback relating to the mental workload can be used for operator training control. In a related, previously filed and commonly assigned U.S. Patent Application Serial No. 10/455,709 filed on June 6, 2003, a system and method for autonomous training was disclosed. Various factors were used with respect to providing feedback regarding training, such as hand controller displacement etc. The parameter of mental workload can be used in a similar manner as a factor used for feedback for an operator training system.

The use of such measurements in training can be advantageous in many situations. For example, suppose that two operators are asked to perform the same task, with one performing the task very easily and within his previous scope of experience, and the other being stretched at the limit of his experience. If the operator that is being stretched in his experience is proud and does not want to exhibit any signs of experiencing difficulty, it would be difficult to measure externally (by observation of face and body movements) any internal mental effort that he is exerting that is higher than the more experienced operator would be exerting. If both operators are successful in completing the task in a similar amount of time, traditional evaluation methods would not find a difference between the performances of the two operators. However, with the ability to measure mental workload according to embodiments of the present invention, it is possible to evaluate the mental workload of a particular operator and to determine whether the operator was at the limit of his abilities and therefore may not be able to perform the task as consistently in the future as the operator for whom the task was simple and required very little mental effort or workload. For example, measurements of mental workload for a particular task and particular operator can be compared to mental workload measurements for the same operator performing different tasks. It is then possible to evaluate whether the particular operator is well suited to perform particular tasks in particular situations. Therefore, it is possible to measure not only performance quality but also operator difficulty according to embodiments of the present invention.

Previous approaches have attempted to use stress measurements in order to determine both performance quality and operator difficulty. Such methods have sometimes been found to be ineffective, as stress can be caused by a variety of factors that are unrelated to the task being performed. For example, systems exist for measuring fatigue for train drivers or truck drivers, such as by measuring skin resistance. However, a drawback is that such measurements only measure the operator's present state and cannot isolate the level of difficulty that the operator is experiencing from other external factors, such as a lack of sleep the night before performing the task. The use of the measurement of Inter Saccadic Interval for measuring mental workload has a much more direct relationship with the actual performance of a task and is less affected by external factors than the methods and systems used in the prior art. Using historical data, it is also possible to determine how close an operator is to his functional limit when undergoing particular mental workload for a task being performed. In order to have the operator become more stable in his execution of the task, the operator may be required to undergo further training, or to repeat previous training, in order to be able to perform the task with less of a demand on mental workload.

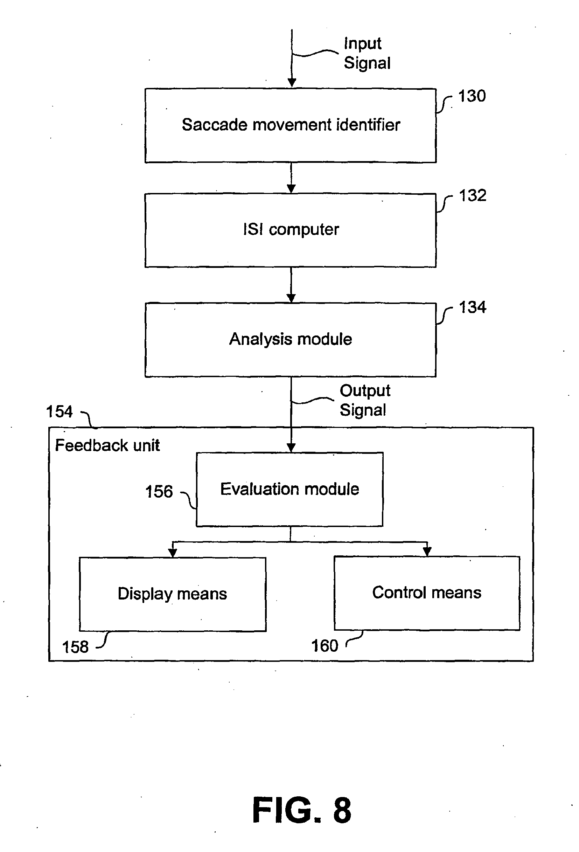

**Fig. 8** is a block diagram of a system incorporating feedback according to an embodiment of the present invention. As in **Fig. 6,** the system includes a saccade movement identifier **130,** or means for identifying saccades in eye movement data. An ISI computer **132,** or means for computing inter-saccadic intervals, is in communication with the saccade movement identifier **130** for computing inter-saccadic intervals in the received eye movement data. An analysis module **134** is provided for determining the mental workload based on the computed inter-saccadic intervals.

In the analysis module **134,** it is possible to calculate the total task workload, as well as to calculate workload levels for different time periods in the task performance. The peak of performance difficulty can also be identified based on the measured value. The output signal can be used as feedback, which can then indicate what type of training may be needed based on the measured values. In such a case, the apparatus can include a feedback unit for modifying a training program based on the determined mental workload. The digital output signal of the analysis module represents parameters of time intervals with different levels of regulation processes in human activity.

In the embodiment of **Fig. 8,** the output of the analysis module (i.e. the measured data) is provided in real time as feedback in the system, such as to a feedback unit **154.** The feedback unit **154** can include an evaluation module **156,** which itself can include simulation/evaluation software. The evaluation module **156** is for evaluating the determined mental workload in relation to stored mental workload data. The stored mental workload data can include average mental workload measurements for a particular task, measurements for a particular operator or group of operators, or any other type of mental workload data, as can be appreciated by one of skill in the art. The feedback information resulting from the processing in the evaluation module can provided to a display means **158,** such as a video display. The feedback information regarding mental workload based on ISI measurements can be displayed to the operator himself. Alternatively, in order not to affect the operator's performance by way of showing the results to him directly, the results can be provided in real time to a third party who is observing the training, and can later be provided to the operator for evaluation of the training exercise. Of course, in some cases, the output signal could be provided directly to the display means **158.** The output of the evaluation module **156** can advantageously be provided to a control means **160.** The control means **160** can be for controlling a device being operated by the operator, and can selectively restrict or allow access to different aspects or modules of the device depending on the results of the feedback from the evaluation module **156.**

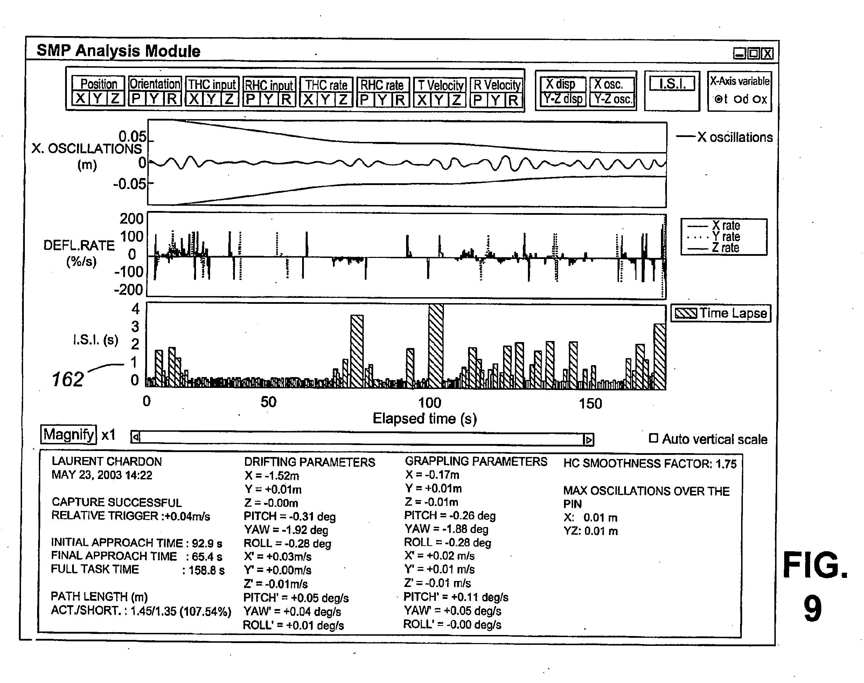

**Fig. 9** is a screen shot of a display showing ISI values for a particular human activity for use as feedback for a training system. This is an example of an implementation of a system according to an embodiment of the present invention using feedback based on measured ISI values, shown in ISI display area **162** in **Fig. 9,** as they relate to mental workload determination. This display and use of the feedback for training is related to the system and method for autonomous training disclosed in related and commonly assigned U.S. Patent Application Serial No. 10/455,709 filed on June 6, 2003.

Embodiments of the present invention can also be used in order to prevent access to a particular task or apparatus if an operator's mental work load measurement is found to be exceeding a particular threshold. The mental workload measurement signal from the evaluation module can be provided to a control means, which can selectively restrict access for an operator based on the comparison of the signal to a threshold value. The threshold can be a general threshold for all operators or can be a personalized threshold for the particular operator. The threshold can be stored in a memory within the control means, or any memory external to, but accessible by, the control means.

Embodiments of the present invention measure the difficulty of a particular person performing a particular task.

<u>Industrial Applicability/Alternative Implementations</u>

Embodiments of the present invention are not limited to the training related applications that are described in detail herein. For example, embodiments of the present invention can be used in relation to usability studies, such as when a new website is being developed and it is desirable to determine which of a plurality of different types of layout is easier to use. This is also applicable to the development of user interfaces for any number of types of computer systems. In all of these case, embodiments of the present invention can be used in a testing, prototype, or market research phase in order to provide objective data on usability. The reliability of various training systems can also be improved by providing feedback based on the mental workload of an operator, in order to reduce the likelihood that an operator will be operating a system when he is at or near the limit of his mental workload capacity. In such cases, the feedback that is measured can be used to trigger a change to an autopilot mode if the operator's mental workload reaches a particular threshold. Embodiments of the present invention can be used in real time performance evaluation and improvement, not only in training scenarios.

Another application of embodiments of the present invention is in the sports and medicine fields. For example, the system can be used in conjunction with people who are deaf or hard of hearing. When these people who are hard of hearing are given certain instructions (whether auditory or visual), the system can be used to measure the mental workload of the person and to determine how difficult it was for that person to acquire and assimilate that information. In sports application, although the system does not provide an indication of physical ability, it does indicate whether the subject is at or near their mental workload limit. In sports as in other areas, when an athlete nears his or her limit, they may push themselves and/or put on a brave face, while they are really mentally nearing their limit. The measurement of ISI can provide an indication of when that limit is first being reached. This can be used in training in order to help the athlete persevere for a longer period of time before their mental limit is reached. The embodiments of the present invention could also be used in academic environments, in order to measure the difficulty that a student is encountering in performing a particular task, such as solving a mathematical problem.

Embodiments of the present invention can also be used in apparatus used to perform lie detector tests. The parameter of Inter Saccadic Intervals can be used in conjunction with other parameters that are typically measured in lie detector tests, in order to increase the reliability of the lie detector test. Typically, one would anticipate that a person answering truthfully would have lower observed ISI since a high mental workload would not be expected. Electroencephalograms are now used for such measurement, but these devices are extremely complicated and the interpretation of their results is subjective and can vary significantly. The indicator of ISI could be more sensitive and more reliable than other factors being used in lie detector tests, though as with any system there may be some false indications. In the lie detector application, the use of digital cameras as a data acquisition means would be advantageous in that the subject would not be aware that such measurements are being taken.

Some previous attempts have used electroencephalogram measurements to attempt to measure mental workload. However, developers of those methods have admitted that those measurements are not reliable or trustworthy in determining mental workload.

The present invention has many potential applications in the scientific, engineering, manufacturing and other fields. The present invention may be used in any one of the following applications: as an instrument for providing assessment of mental workload of human performance in human factors studies and usability analysis; as a tool for measuring human training and proficiency in simulators and real conditions; as a part of an electronic safety system to detect activity degradation due to high operator activity complexity in control tasks; and as a tool for situation awareness and decision making measurement. Particularly, the present invention can be advantageously employed for human operator activity assessment at air jet plane computer simulators during laboratory experimental research.

The present invention has particular applications to highly automated control systems in which human operator activity is connected with any of the following characteristics: complex, responsible decision making; multiple parameter information processes; logical and spatial images; flexible planning; anticipation and situation awareness.

An example of such an application is the assessment of the activity of an operator in computer control systems for energy stations, technology processes, aviation, space and naval transport vehicles. Another such application is the assessment of activity of personal computer users.

In addition, the present invention may be used in estimation of mental workload of human performance in education, medicine and sport training. Particularly, the present invention can advantageously be employed for assessment of mood state levels in extreme conditions of operator performance. Such extreme conditions can be simulated in laboratory experiments, and can include 3 days sleep deprivation, and experiments of naupathia (motion sickness) at sea in conditions of rolling and pitching. Embodiments of the present invention can also be used in the evaluation of the level of human properties and peculiarities and abnormal states in mental, cognitive and regulation processes in experiments of fatigue during sport exercises and psychological testing in laboratory research.

As mentioned earlier, embodiments of the present invention can use a mental workload measurement (determined according to any one of the methods described earlier) as a means of determining a number of useful indications, including but not limited to: human performance based on the measuring of ISI in eye movements; human performance dynamics based on measuring changes in ISI; a total mental workload of human performance; a total and current mental workload of human performance; a current mental workload of human performance in real-time, e.g. on-line; human proficiency level in simulators and real conditions; interface perfection of a man-machine system; software usability; complexity of human controlled procedures; presenting feedback about the current mental workload of human performance to human in real-time, e.g. on-line; changing a human controlled system mode or training scenarios in response to the current mental workload of human performance; human proficiency properties; level of human mood states in extreme conditions of operator performance; level of human properties and peculiarities in mental, cognitive and regulative processes in any one of education, medicine, sport training, and the like.

The above-described embodiments of the present invention are intended to be examples only. Alterations, modifications and variations may be effected to the particular embodiments by those of skill in the art without departing from the scope of the invention, which is defined by the claims appended hereto.

**Claims**

1. A method of measuring a mental workload for a human based on rapid eye movements, comprising:

   identifying saccades in received eye movement data;
   computing inter-saccadic intervals for the received eye movement data; and
   determining the mental workload based on the computed inter-saccadic intervals.

2. The method of claim 1 wherein the step of identifying includes an automatic saccade determination scheme.

3. The method of claim 2 wherein the automatic saccade determination scheme is a velocity detection method.

4. The method of claim 3 wherein the velocity detection method includes:

   computing eye movement speed based on the eye movement data;
   comparing computed speed to threshold level; and
   determining presence of saccade if computed speed is above the threshold.

5. The method of claim 4 wherein the step of computing eye movement speed includes:

   smoothing the eye movement data with a low-pass digital filter to remove noise; and numerically differentiating the filter output.

6. The method of claim 1 wherein the step of computing inter-saccadic intervals includes:

   identifying a boundary of a saccade;
   computing an inter-saccadic interval between the boundary of the current saccade and a boundary of a next saccade; and
   storing parameter values in a memory.

7. The method of claim 6 wherein the step of identifying the boundary of the saccade comprises identifying a start of the saccade in response to a measured speed of eye movement being more than a threshold.

8. The method of claim 6 wherein the step of identifying the boundary of the saccade comprises identifying an end of the saccade in response to a measured speed of eye movement being less than a threshold.

9. The method of claim 1 further comprising the step of receiving the eye movement data.

10. The method of claim 1 further comprising the step of using the determined mental workload as feedback for training.

11. The method of claim 1 further comprising the step of identifying a peak mental workload value for a period of time.

12. The method of claim 1 further comprising the step of determining a real-time mental workload value.

13. An apparatus for measuring a mental workload for a human based on rapid eye movements, comprising:

   means for identifying saccades in eye movement data;
   means for computing inter-saccadic intervals in the received eye movement data; and an analysis module for determining the mental workload based on the computed intersaccadic intervals.

14. The apparatus of claim 13 further comprising an ocular sensor for obtaining the eye movement data.

**15.** The apparatus of claim 14 wherein the ocular sensor includes at least one video ocular sensor.

**16.** The apparatus of claim 15 wherein the video ocular sensor is a digital video camera.

**17.** The apparatus of claim 14 wherein the ocular sensor includes at least one electric ocular sensor.

**18.** The apparatus of claim 17 wherein the electric ocular sensor includes:

electrodes;
an amplifier; and
an analog/digital (A/D) converter.

**19.** The apparatus of claim 13 further comprising a feedback unit for modifying a training program based on the determined mental workload.

**20.** The apparatus of claim 19 wherein the feedback unit includes an evaluation module for evaluating the determined mental workload in relation to stored mental workload data.

**21.** The apparatus of claim 20 wherein the feedback unit includes a control means for controlling access to a device being operated by an operator, based on an output of the evaluation module.

**22.** The apparatus of claim 19 wherein the feedback unit includes a display means for displaying mental workload data based on the determined mental workload.

**23.** A computer-readable medium including statements and instructions which, when executed by a computer, cause the computer to perform the steps of:

identifying saccades in received eye movement data;
computing inter-saccadic intervals for the received eye movement data; and
determining the mental workload based on the computed inter-saccadic intervals.

**24.** A signal embodied on a carrier wave, the signal comprising:

a first data segment for identifying saccades in received eye movement data;
a second data segment for computing inter-saccadic intervals for the received eye movement data; and
a third data segment for determining the mental workload based on the computed inter-saccadic intervals.

**25.** The method of claim 1 wherein the step of determining the mental workload includes assigning a selected computed inter-saccadic interval to a task complexity level.

**26.** The method of claim 25 wherein the step of assigning includes comparing the selected computed inter-saccadic interval to an upper boundary of a range, the upper boundary representing an upper boundary of mental workload for the task complexity level.

**Patentansprüche**

**1.** Verfahren zur Messung der geistigen Arbeitsbelastung eines Menschen auf Grundlage rascher Augenbewegungen (REM), umfassend:

das Erkennen von Sakkaden aus den erhaltenen Daten der Augenbewegung;
das Berechnen von Intervallen zwischen den Sakkaden aus den erhaltenen Daten der Augenbewegung; und
das Bestimmen der geistigen Arbeitsbelastung auf der Grundlage der berechneten Intervalle zwischen den Sakkaden .

**2.** Verfahren nach Anspruch 1, wobei der Schritt des Erkennens eine automatische Maßnahme zur Bestimmung der Sakkaden umfasst.

3. Verfahren nach Anspruch 2, wobei die automatische Maßnahme zur Bestimmung der Sakkaden ein Geschwindigkeitsmessverfahren ist.

4. Verfahren nach Anspruch 3, wobei das Geschwindigkeitsmessverfahren umfasst:

   das Berechnen der Geschwindigkeit der Augenbewegung auf der Grundlage der Daten der Augenbewegung;
   das Vergleichen der berechneten Geschwindigkeit mit einem Schwellenwert; und
   das Bestimmen des Vorliegens einer Sakkade, wenn die berechnete Geschwindigkeit über dem Schwellenwert liegt.

5. Verfahren nach Anspruch 4, wobei der Schritt des Berechnens der Geschwindigkeit der Augenbewegung umfasst:

   das Glätten der Daten der Augenbewegung mit einem digitalen Tiefpass-Filter zur Rausch-Unterdrückung; und eine numerische Differentiation der Ausgabe desFilters.

6. Verfahren nach Anspruch 1, wobei der Schritt zum Berechnen der Intervalle zwischen den Sakkaden umfasst:

   das Erkennen einer Grenze einer Sakkade;
   das Berechnen eines Intervalls zwischen der Grenze der aktuellen Sakkade und der Grenze einer folgenden Sakkade; und
   das Speichern von Parameterwerten in einem Speicher.

7. Verfahren nach Anspruch 6, wobei der Schritt zur Erkennung der Grenze der Sakkade das Erkennen des Beginns der Sakkade als Antwort auf eine gemessene Geschwindigkeit der Augenbewegung, die über einem Schwellenwert liegt, umfasst.

8. Verfahren nach Anspruch 6, wobei der Schritt zur Erkennung der Grenze der Sakkade die Bestimmung eines Endes der Sakkade als Antwort auf eine gemessene Geschwindigkeit der Augenbewegung, die unter einem Schwellenwert liegt, umfasst.

9. Verfahren nach Anspruch 1, das zusätzlich umfassend einen Schritt zum Empfang der Daten der Augenbewegung:

10. Verfahren nach Anspruch 1, das zusätzlich umfassend einen Schritt der Verwendung der ermittelten geistigen Arbeitsbelastung als Trainingsfeedback.

11. Verfahren nach Anspruch 1, das zusätzlich umfassend einen Schritt zur Ermittlung eines Höchstwertes der geistigen Arbeitsbelastung für eine Zeitspanne.

12. Verfahren nach Anspruch 1, das zusätzlich umfassend einen Schritt zur Ermittlung eines Wertes der geistigen Arbeitsbelastungs in Echtzeit.

13. Vorrichtung zur Messung der geistigen Arbeitsbelastung eines Menschen auf der Grundlage rascher Augenbewegungen (REM), umfassend:

   Mittel zur Erkennung von Sakkaden aus den erhaltenen Daten der Augenbewegung;
   Mittel zum Berechnen von Intervallen zwischen den Sakkaden aus den erhaltenen Daten der Augenbewegung; und ein Analysemodul zur Bestimmung der geistigen Arbeitsbelastung auf Grundlage der berechneten Intervalle zwischen den Sakkaden.

14. Vorrichtung nach Anspruch 13, das zusätzlich umfassend einen Okularsensor zum Erhalt der Daten der Augenbewegung.

15. Vorrichtung nach Anspruch 14, wobei der Okularsensor wenigstens einen Video-Okularsensor umfasst.

16. Vorrichtung nach Anspruch 15, wobei der Video-Okularsensor eine digitale Videokamera ist.

17. Vorrichtung nach Anspruch 14, wobei der Okularsensor wenigstens einen elektrischen Okularsensor aufweist.

**18.** Vorrichtung nach Anspruch 17, wobei der elektrische Okularsensor umfasst:

Elektroden;
einen Verstärker; und
einen Analog/digital (A/D)-Wandler.

**19.** Vorrichtung nach Anspruch 13, zusätzlich umfassend eine Rückmeldeeinheit zum Modifizieren eines Trainingsprogramms auf der Grundlage der bestimmten geistigen Arbeitsbelastung.

**20.** Vorrichtung nach Anspruch 19, wobei die Rückmeldeeinheit ein Auswertemodul zur Auswertung der bestimmten geistigen Arbeitsbelastung in Bezug auf gespeicherte geistige Arbeitsbelastungsdaten umfasst.

**21.** Vorrichtung nach Anspruch 20, wobei die Rückmeldeeinheit Steuermittel zur Steuerung des Zugangs zu einer Vorrichtung aufweist, die von einer Bedienperson basierend auf einer Ausgabe des Auswertemoduls bedienbar sind.

**22.** Vorrichtung nach Anspruch 19, wobei die Rückmeldeeinheit Anzeigemittel zur Anzeige von Daten der geistigen Arbeitsbelastung auf der Grundlage der bestimmten geistigen Arbeitsbelastung umfasst.

**23.** Computerlesbares Medium, umfassend Anweisungen und Instruktionen, die bei Ausführung mittels eines Computers bewirken, daß der Computer folgende Schritte ausführt:

Erkennen von Sakkaden in erhaltenen Daten der Augenbewegung;
Berechnen von Intervallen zwischen den Sakkaden aus den erhaltenen Daten der Augenbewegung; und
Bestimmen der geistigen Arbeitsbelastung auf der Grundlage der berechneten Intervalle zwischen den Sakkaden.

**24.** Signal ausgebildet auf einer Trägerwelle, wobei das Signal umfasst:

ein erstes Datensegment zur Erkennung von Sakkaden aus den erhaltenen Daten der Augenbewegung;
ein zweites Datensegment zur Berechnung von Intervallen zwischen den Sakkaden aus den erhaltenen Daten der Augenbewegung; und
ein drittes Datensegment zur Bestimmung der geistigen Arbeitsbelastung auf der Grundlage der berechneten Intervalle zwischen den Sakkaden.

**25.** Verfahren nach Anspruch 1, wobei der Schritt zur Bestimmung der geistigen Arbeitsbelastung eine Zuordnung eines ausgewählten berechneten Intervalls zwischen den Sakkaden zu einem Schwierigkeitsgrad der Aufgabe umfasst.

**26.** Verfahren nach Anspruch 25, wobei der Schritt der Zuordnung einen Vergleich des ausgewählten berechneten Intervalls zwischen den Sakkaden mit einer Obergrenze eines Bereiches umfasst, wobei die Obergrenze eine Obergrenze der geistigen Arbeitsbelastung für den Schwierigkeitsgrad der Aufgabe ist.


**Revendications**

**1.** Procédé de mesure d'une charge de travail mental pour un humain sur la base de mouvements rapides des yeux, comprenant :

l'identification de saccades dans des données de mouvements des yeux reçues ;
le calcul d'intervalles inter-saccades pour les données de mouvements des yeux reçues ; et
la détermination de la charge de travail mental sur la base des intervalles inter-saccades calculés.

**2.** Procédé selon la revendication 1 dans lequel l'étape d'identification inclut un programme automatique de détermination de saccades.

**3.** Procédé selon la revendication 2 dans lequel le programme automatique de détermination de saccades est un procédé de détection de vélocité.

**4.** Procédé selon la revendication 3 dans lequel le procédé de détection de vélocité inclut :

le calcul de la vitesse de mouvements des yeux sur la base des données de mouvements des yeux ;
la comparaison d'une vitesse calculée avec un niveau seuil ; et
la détermination de la présence d'une saccade si la vitesse calculée est supérieure au seuil.

**5.** Procédé selon la revendication- 4 dans lequel l'étape de calcul de la vitesse de mouvements des yeux inclut :

le lissage des données de mouvements des yeux avec un filtre numérique passe-bas pour supprimer le bruit ; et
la différentiation numérique de la sortie de filtre.

**6.** Procédé selon la revendication 1 dans lequel l'étape de calcul des intervalles inter-saccades inclut :

l'identification d'une limite d'une saccade ;
le calcul d'un intervalle inter-saccades entre la limite de la saccade actuelle et une limite d'une saccade suivante ; et
le stockage de valeurs de paramètres dans une mémoire.

**7.** Procédé selon la revendication 6 dans lequel l'étape d'identification de la limite de la saccade comprend l'identification d'un début de la saccade en réponse à une vitesse mesurée du mouvement des yeux étant supérieure à un seuil.

**8.** Procédé selon la revendication 6 dans lequel l'étape d'identification de la limite de la saccade comprend l'identification d'une fin de la saccade en réponse à une vitesse mesurée du mouvement des yeux étant inférieure à un seuil.

**9.** Procédé selon la revendication 1 comprenant en outre l'étape de réception des données de mouvements des yeux.

**10.** Procédé selon la revendication 1 comprenant en outre l'étape d'utilisation de la charge de travail mental déterminée en tant que retour d'information pour une formation.

**11.** Procédé selon la revendication 1 comprenant en outre l'étape d'identification d'une valeur de pic de charge de travail mental pour une période de temps.

**12.** Procédé selon la revendication 1 comprenant en outre l'étape de détermination d'une valeur de charge de travail mental en temps réel.

**13.** Appareil pour mesurer une charge de travail mental pour un humain sur la base de mouvements rapides des yeux, comprenant :

un moyen pour identifier des saccades dans des données de mouvements des yeux ;
un moyen pour calculer des intervalles inter-saccades dans les données de mouvements des yeux reçues ; et
un module d'analyse pour déterminer la charge de travail mental sur la base des intervalles inter-saccades calculés.

**14.** Appareil selon la revendication 13 comprenant en outre un capteur oculaire pour obtenir les données de mouvements des yeux.

**15.** Appareil selon la revendication 14 dans lequel le capteur oculaire inclut au moins un capteur oculaire vidéo.

**16.** Appareil selon la revendication 15 dans lequel le capteur oculaire vidéo est une caméra vidéo numérique.

**17.** Appareil selon la revendication 14 dans lequel le capteur oculaire inclut au moins un capteur oculaire électrique.

**18.** Appareil selon la revendication 17 dans lequel le capteur oculaire électrique inclut :

des électrodes ;
un amplificateur ; et
un convertisseur analogique/numérique (A/N).

**19.** Appareil selon la revendication 13 comprenant en outre une unité de retour d'information pour modifier un programme de formation sur la base de la charge de travail mental déterminée.

**20.** Appareil selon la revendication 19 dans lequel l'unité de retour d'information inclut un module d'évaluation pour évaluer la charge de travail mental déterminée en relation avec des données de charge de travail mental stockées.

**21.** Appareil selon la revendication 20 dans lequel l'unité de retour d'information inclut un moyen de commande pour commander l'accès à un dispositif utilisé par un opérateur, sur la base d'une sortie du module d'évaluation.

**22.** Appareil selon la revendication 19 dans lequel l'unité de retour d'information inclut un moyen d'affichage pour afficher des données de charge de travail mental sur la base de la charge de travail mental déterminée.

**23.** Support lisible par un ordinateur incluant des relevés et des instructions qui, lorsqu'elles sont exécutées par un ordinateur, font que l'ordinateur exécute les étapes :

d'identification de saccades dans des données de mouvements des yeux reçues ;
de calcul d'intervalles inter-saccades pour les données de mouvements des yeux reçues ; et
de détermination de la charge de travail mental sur la base des intervalles inter-saccades calculés.

**24.** Signal intégré sur une onde porteuse, le signal comprenant :

un premier segment de données pour identifier des saccades dans des données de mouvements des yeux reçues ;
un deuxième segment de données pour calculer des intervalles inter-saccades pour les données de mouvements des yeux reçues ; et
un troisième segment de données pour déterminer la charge de travail mental sur la base des intervalles inter-saccades calculés.

**25.** Procédé selon la revendication 1 dans lequel l'étape de détermination de la charge de travail mental inclut l'attribution d'un intervalle inter-saccades calculé sélectionné à un niveau de complexité de tâche.

**26.** Procédé selon la revendication 25 dans lequel l'étape d'attribution inclut la comparaison de l'intervalle inter-saccades calculé sélectionné à une limite supérieure d'une plage, la limite supérieure représentant une limite supérieure de charge de travail mental pour le niveau de complexité de tâche.

100

```
┌─ ─ ─ ─ ─ ─ ─ ─ ─ ─ ─ ─ ─ ─ ─ ─ ┐
│                                  │   108
│        Receive eye movement data │
│                                  │
└─ ─ ─ ─ ─ ─ ─ ─ ─ ─ ─ ─ ─ ─ ─ ─ ┘
```

Receive eye movement data — 108

Identify saccades in received eye movement data — 102

Compute inter-saccadic intervals for the received eye movement data — 104

Determine mental workload based on the computed inter-saccadic intervals — 106

Use the determined mental workload value as feeedback for training — 110

# FIG. 1

102

Identify saccades in received eye movement data

112

| Compute eye movement speed based on the eye movement data |
|---|

114

| Compare the computed speed to a threshold value |
|---|

116

| Determine the presence of a saccade if the computed speed is above the threshold |
|---|

118

| Smooth eye movement data with low-pass digital filter to remove noise |
|---|

120

| Numerically differentiate the filter output |
|---|

## FIG. 2

104

Compute inter-saccadic intervals for the received eye movement data

124

122

| Identify a boundary of a saccade |
|---|

| Compute interval between the boundary of the saccade and the boundary of a previous saccade |
|---|

126

| Store parameter values in memory |
|---|

## FIG. 3

**FIG. 4**

**FIG. 5**

AUGUST 22
V=1.094.5-11191.7  S=6.14-6.68 VINT.S

ISI, SEC TAXONS

**FIG. 6**

FIG. 7

Input
Signal

Saccade movement identifier — 130

ISI computer — 132

Analysis module — 134

Output
Signal

154 —

Feedback unit

156 — Evaluation module

Display means

Control means

158 —

160 —

# FIG. 8

FIG. 9

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 6090051 A, Marshall
- US 6102870 A
- US 455709 A
- US 45570903 A

**Non-patent literature cited in the description**

- **YU. BERGER ; A. LUUK ; T. MOGOM.** A comparative analysis of algorithms for detection of saccades. *Automated Real Time Systems for Ergonomic Studies,* 1988, 271-274
- **YU. BERGER ; T. MOGOM.** The effect of Ways of Processing a Signal upon Parameters of Saccadic Eye movement. *Automated Real Time Systems for Ergonomic Studies,* 1988, 274-277
- **A.M. BOUKADOUM ; P.Y. KTONAS.** EOG-based recording and automated detection of sleep rapid eye movements: a critical review, and some recommendations. *Psychophysiology,* 1986, vol. 23 (5), 598-611